# NEUE EUROPÄISCHE PATENTSCHRIFT

(11) **EP 0 809 805 B2**
(45) Veröffentlichungstag und Bekanntmachung des Hinweises auf die Entscheidung über den Einspruch: **26.03.2003**
(45) Hinweis auf die Patenterteilung: 02.06.1999
(21) Anmeldenummer: 96901792.0
(22) Anmeldetag: 07.02.1996
(51) Int. Cl.: G01N 33/564, G01N 33/567, G01N 33/78, G01N 33/543

(54) **VERWENDUNG VON POLYKLONALEN HUMANEN ANTI-HTG-AUTOANTIKÖRPERN ALS REAGENZ FÜR DIE KLINISCHE DIAGNOSTIK VON SCHILDDRÜSEN-AUTOIMMUNERKRANKUNGEN SOWIE REAGENZIENSATZ FÜR EINE BESTIMMUNG VON ANTI-HTG-AUTOANTIKÖRPERN IN PATIENTENSEREN**
USE OF POLYCLONAL HUMAN ANTI-HTG AUTO-ANTIBODIES AS A REAGENT FOR THE CLINICAL DIAGNOSIS OF THYROID AUTO-IMMUNE DISEASES AND REAGENT ADDITIVE FOR DETECTING ANTI-HTG AUTO-ANTIBODIES IN PATIENTS' SERA
UTILISATION D'AUTOANTICORPS ANTI-HTG POLYCLONAUX HUMAINS COMME REACTIFS POUR LE DIAGNOSTIC CLINIQUE D'AFFECTIONS AUTO-IMMUNES DE LA THYROIDE ET JEU DE REACTIFS SERVANT A DETECTER LES AUTOANTICORPS ANTI-HTG DANS LES SERUMS DE PATIENTS

(30) Priorität: 16.02.1995 DE 19505266
(43) Veröffentlichungstag der Anmeldung: 03.12.1997
(73) Patentinhaber: B.R.A.H.M.S DIAGNOSTICA GMBH, 12099 Berlin (DE)
(72) Erfinder: BERGMANN, Andreas, D-12351 Berlin (DE)
(74) Vertreter: Andrae, Steffen, Dr.
(86) Internationale Anmeldenummer: EP9600514
(87) Internationale Veröffentlichungsnummer: WO96025668

(56) Entgegenhaltungen:
- WO-A-92/21031
- DE-C- 4 120 412
- GB-A- 2 265 713
- PL-B- 162 445
- HENNING test anti-Tg, Arbeitsanleitung, BRAHMS Diagnostica GmbH, aktueller Stand 25.01.1999
- HENNING Berlin, Sonderheft II, Band 10, November 1989, Seiten 90-102, Aktuelle Endokrinologie und Stoffwechsel.

## Beschreibung

Die vorliegende Erfindung betrifft den Nachweis von Antikörpern in biologischen Flüssigkeiten mit Hilfe immunologischer Bestimmungsverfahren, die mit Hilfe von Reagenziensätzen (Kits) für die klinische Diagnostik durchgeführt werden, die in industriellem Maßstab von Diagnostikaherstellern erstellt und vertrieben werden. Insbesondere betrifft die vorliegende Erfindung den Nachweis von Autoantikörpern, und zwar insbesondere von Autoantikörpern gegen die Autoantigene der Schilddrüse, von denen wiederum im Rahmen der vorliegenden Erfindung die Bestimmung von humanen Autoantikörpern gegen Thyreoglobulin (hTg) in einem humanen Serum oder Plasma im Vordergrund steht.

Thyreoglobulin ist eines der Schilddrüsen-Autoantigene und stellt ein hochmolekulares Protein dar, das aus zwei identischen glykosylierten Untereinheiten mit einem Molekulargewicht von jeweils etwa 330 kD besteht (vgl. z.B. J. Furmaniak und B. Rees Smith, in: Autoimmunity, 1990, Vol.7, S. 63-80). Thyreoglobulin ist ein Hauptbestandteil des Schilddrüsenkolloids und stellt den Vorläufer der Schilddrüsenhormone Triiodthyronin (T₃) und Thyroxin (T₄) dar. Als Vorstufe zur Synthese der genannten Schilddrüsenhormone wird unter katalytischer Einwirkung des Enzyms Schilddrüsenperoxidase (TPO) ungebundenes zirkulierendes Jod beziehungsweise Jodid in Tyrosylreste oder Jodtyrosinreste des Tg eingebaut. Aus dem jodierten Thyreoglobulin werden dann die Schilddrüsenhormone T₃ und T₄ freigesetzt, und zwar durch hormonale Steuerungsmechanismen, die auf den Gehalt an T₃ beziehungsweise T₄ im Blut reagieren.

Bei Schilddrüsen-Autoimmunerkrankungen, und zwar insbesondere bei der zu einer Hypothyreose führenden destruktiven Hashimoto-Thyreoiditis, werden im Blut der Patienten neben Autoantikörpern gegen Schilddrüsenperoxidase in erheblichen Mengen Autoantikörper gegen Thyreoglobulin festgestellt (im folgenden werden derartige Antikörper stets als "anti-hTg-Autoantikörper" bezeichnet). Dem Nachweis und der quantitativen Bestimmung derartiger anti-hTg-Autoantikörper kommt somit in der klinischen Diagnostik von Schilddrüsenerkrankungen eine erhebliche Bedeutung zu.

Die Bestimmung von anti-hTg-Autoantikörpern in Patientenseren ist nach verschiedenen Techniken möglich, unter anderem auch nach immunologischen Bestimmungsverfahren. Bei einem im Handel befindlichen derartigen Bestimmungsverfahren (HENNINGtest®anti-Tg) wird dabei so vorgegangen, daß in den Teströhrchen vorverdünnte Serumproben gleichzeitig mit einem markierten Tg (als Tracer) und einer Protein-A-Suspension inkubiert werden. Sind anti-hTg-Autoantikörper in der Probe vorhanden, so reagieren sie mit dem als Tracer zugesetzten Antigen hTg. Gleichzeitig bindet das in Form von Teilchen einer suspendierten Festphase vorliegende Protein-A unspezifisch an die Fc-Untereinheiten der Antikörper, und es entsteht ein Sandwichkomplex. Durch eine Zentrifugation werden die Teilchen der Festphasensuspension mit sämtlichen daran gebundenen Molekülen in ein Sediment überführt, und der Überstand mit dem ungebundenen Tracer wird entfernt. Je größer die Menge der Autoantikörper in der Probe ist, desto mehr Tracer wird gebunden. Damit ist die Konzentration der anti-hTg-Autoantikörper direkt proportional der im Sediment nachweisbaren Tracermenge, zum Beispiel bei radioaktiver Markierung der im Sediment meßbaren Radioaktivität.

Durch Verwendung des unspezifischen Bindungspartners Protein-A, mit dessen Hilfe unterschiedslos ganz Klassen von IgG-Antikörpern gebunden werden können, und durch die gleichzeitige Verwendung von markiertem Thyreoglobulin als Tracer werden bei dem beschriebenen Bestimmungsverfahren alle anti-hTg-Autoantikörper erfaßt. Da bekannt ist, daß in Patientenseren auch anti-hTg-Autoantikörper vorkommen können, die nicht direkt mit einer klinisch manifesten Schilddrüsen-Autoimmunerkrankung in Verbindung gebracht werden können, wären aber an sich auch Bestimmungsverfahren von Interesse, die selektiver auf solche anti-hTg-Autoantikörper ansprechen, die direkt und ursächlich mit klinisch manifesten Schilddrüsenerkrankungen wie zum Beispiel der Hashimoto-Thyreoiditis verknüpft sind. Außerdem kann es als ein gewisser Nachteil des beschriebenen bekannten Verfahrens angesehen werden, daß es einen Zentrifugationsschritt erfordert. Zur Bestimmung von anti-TPO-Autoantikörpern, die sehr häufig parallel zur Bestimmung von anti-hTg-Autoantikörpern durchgeführt wird, steht jedoch ein anderes, als kompetitives Bestimmungsverfahren arbeitendes Verfahren zur Verfügung, das als Coated-Tube-Verfahren ohne einen Zentrifugationsschritt auskommt (DYNOtest® anti-TPO; vgl. bezüglich des Verfahrensprinzips DE 41 20 412 Cl). Die gleichzeitige Durchführung von zwei Bestimmungsverfahren, die mit unterschiedlichen Handhabungsschritten verbunden sind, stellt jedoch in der klinischen Laborpraxis einen Nachteil dar. Es wäre somit an sich wünschenswert, auch die Bestimmung von anti-hTg-Autoantikörpern nach einem Bestimmungsverfahren durchführen zu können, das nach der Coated-Tube-Technik arbeitet und zu den kompetitiven Bestimmungsverfahren gehört und bei dem sich die Anwesenheit der zu bestimmenden Antikörper in der untersuchten Serumprobe als Verminderung der Bindung des markierten Reagenz (Tracers) an die Wände eines Teströhrchens äußert.

Während in Falle der Bestimmung von anti-TPO-Autoantikörpern die Epitope des Antigens hTPO im wesentlichen bekannt sind und ein für eine kompetitive Bestimmung geeigneter Satz monoklonaler Antikörper gefunden werden kann (vgl. Jean Ruf, Marie-Elisabeth Toubert, Barbara Czarnocka, Josee-Martine Durand-Gorde, Mireille Ferrand und Pierre Carayon, in: Endocrinology, Vol. 125, No.3, S. 1211-1218, 1989), stößt die Schaffung vergleichbarer kompetitiver Bestimmungsverfahren in Fällen, bei denen die Antigene sehr groß und/oder bezüglich ihrer an der Immunreaktion beteiligten Epitope ungenügend charakterisiert oder sogar unbekannt sind, bisher auf grundsätzliche Probleme. Derartige Fälle sind sehr zahlreich, und als Beispiele für derartige Antigene können genannt werden die Glutamatdecarboxylase (Diabetes Typ I), der Acetylcholinrezeptor (Myasthenia gravis), das sogenannte "myelin basic protein" (Multiple Sklerose), der TSH-Rezeptor und insbesondere das Thyreoglobulin (hTg).

Versucht man, zur Bestimmung von anti-hTg-Autoantikörpern ein kompetitives Bestimmungsverfahren zu entwickeln, das dem o.g. beschriebenen Verfahren zur Bestimmung von anti-TPO-Autoantikörpern entspricht, stößt man auf Schwierigkeiten, die mit den Besonderheiten des als Antigen wirkenden Thyreoglobulins beziehungsweise der gegen Thyreoglobulin gebildeten Antikörperpopulationen zu tun haben. Wie eingangs ausgeführt wurde, ist humanes Thyreoglobulin (hTg) ein sehr großes proteinisches Antigen mit einer sehr hohen Anzahl von potentiellen antigenen Determinanten (Epitopen), die dazu führen, daß man bei der Verwendung von humanem Thyreoglobulin zur Erzeugung heterologer Antikörper durch direkte Immunisierung eines Tiers eine Population polyklonaler Antikörper erhält, von denen festgestellt wurde, daß sie sich gegen eine große Anzahl verschiedener Epitope des humanen Thyreoglobulins richten. Untersuchungen der letzten Jahre haben jedoch gezeigt, daß die bei Autoimmunerkrankungen gebildeten anti-hTg-Autoantikörper offensichtlich nur gegen eine beschränkte Anzahl von Epitopen auf der Thyreoglobulinoberfläche gebildet werden (J. Furmaniak und B. Rees Smith, in: Autoimmunity, 1990, Vol.7, S. 63-80; J.Ruf, Mireille Henry, Catherine De Micco, P. Carayon, in: Thyroglobulin and Thyroglobulin Antibodies in the Follow-up of Thyroid Cancer and Endemic Goiter (Hüfner, M., und Reiners, C., Herausg.), S.21-31, 1987, G.T.Verlag Stuttgart, New York; C.T.J. Chan, P.G.H. Byfield, R.L. Himsworth und P.Shepherd, in: Clin.exp.Immunol. (1987) 70, S.516-523; S.M. McLachlan, U. Feldt-Rasmussen, E.T. Young, S.L. Middleton, M. Blichert-Toft, K. Siersboek-Nielsen, J. Date, D. Carr, F. Clark und B. Rees Smith, in: Clinical Endocrinology (1987), 26, S.335-346; Q. Dong, M. Ludgate und G. Vassart, in: Journal of Endocrinology (1989) 122, S.169-176; M.E. Devey, K.M. Bleasdale-Barr, S.M. McLachlan, J. Bradbury, F. Clark und E.T. Young, in: Clin.exp.Immunol. (1989), 77, S.191-195; N. Fukuma, S.M. McLachlan, V.B. Petersen, P. Kau, J. Bradbury, M. Devey, K. Bleasdale, P. Grabowski und B. Rees Smith, in: Immunology 1989, 67, S.129-131; N. Fukuma, S.M. McLachlan, V.B. Petersen, K. Beever und B. Rees Smith, in: Autoimmunity, 1990, Vol.6, S.37-45; Shannon L. Gleason, Patricia Gearhardt, Noel R. Rose und Rudolf C. Kuppers, in: The Journal of Immunology, Vol. 145, No. 6, S.1768-1775, 1990; Herbert S. Bresler, C. Lynne Burek und Noel R. Rose, in: Clinical Immunology and Immunopathology, Vol.54, S.64-75, (1990); Herbert S. Bresler, C. Lynne Burek, William H. Hoffman und Noel R. Rose, in: Clinical Immunology and Immunopathology, Vol.54, S.76-86, (1990); Mireille Henry, Yves Malthièry, Eric Zanelli und Bernadette Charvet, in: The Journal of Immunology, Vol. 145, No.11, S.3692-3698, 1990; Shigeki Sakata, Toru Ogawa, Yasuyoshi Kimata, Hiroshi Takuno, Hiroshi Maekawa, Masafumi Matsuda, Osamu Tarutani und Kenji Okuda, in: Molecular and Cellular Endocrinology, 79, (1991), S.93-98; Gilles Dietrich, Martine Piechaczyk, Bernard Pau und Michel D. Kazatchkine, in: Eur.J.Immunol. 1991, 21, S.811-814; Yves Malthièry, Mireille Henry und Eric Zanelli, in: FEBS Letters, Vol. 279, No.2, S.190-192 (1991); E. Schulz, G. Benker, H. Bethäuser, L. Stempka und M. Hüfner, in: J. Endocrinol. Invest 15, S. 25-30, 1992; Asmae Alami Harchali, Paul Montagne, Marie L. Cuillière, Majida Bouanani, Bernard Pau und Jean Duheille, in: Clin.Chem. Vol.38, No.9, S.1859-1864 (1992); B. Mallet, P.J. Lejeune, J. Ruf, M. Piechaczyk, C. Marriq und P. Carayon, in: Molecular and Cellular Endocrinology, 88, (1992), S.89-95; J.M.Hexham, J.Furmaniak, C.Pegg, D.R.Burton und B.Rees Smith, in: Autoimmunity, 1992, Vol.12, S.135-141; Rudolf C. Kuppers, Ingrid M. Outschoorn, Robert G. Hamilton, C. Lynne Burek und Noel R. Rose, in: Clinical Immunology and Immunopathology, Vol. 67, No.1, S.68-77, 1993; Peter J. Delves, Sandra M. McLachlan, Elizabeth Drewe, Nao Fukuma, Vaughan B. Petersen und Bernard Rees Smith, in: Journal of Autoimmunity (1993), 1, S.77-91; Patrizio Caturegli, Stefano Mariotti, Rudolf C. Kuppers, C. Lynne Burek, Aldo Pinchera und Noel R. Rose, in: Autoimmunity, 1994, Vol. 18, S.41-49). Aufgrund der Komplexität des Problems ist ein vollständiges Epitop-Mapping für hTg bisher noch nicht gelungen, wobei die Situation dadurch erschwert wird, daß es sich gezeigt hat, daß die bei Autoimmunerkrankungen gebildeten Autoantikörper anscheinend zu den konformativen Antikörpern gehören, was den Versuch eines Epitop-Mappings unter Verwendung monoklonaler anti-hTg-Antikörper tierischen Ursprungs weiter erschwert und ein Mapping der relevanten Epitope unter Verwendung rekombinanter, sequentieller Antikörper verhindert (Q. Dong, M. Ludgate und G. Vassart, in: Journal of Endocrinology (1989) 122, S.169-176). Durch Immunisierung mit Hilfe von hTg in den Seren von Tieren erhaltene polyklonale heterologe IgG-Populationen entsprechen somit nicht den bei Autoimmunerkrankungen auftretenden anti-hTg-Autoantikörperpopulationen und sind daher offensichtlich für eine Verwendung in kompetitiven immunologischen Bestimmungsverfahren wenig oder nicht geeignet.

Andererseits hat sich gezeigt, daß auch der Versuch einer Verwendung monoklonaler anti-hTg-Antikörper in kompetitiven Bestimmungsverfahren zur Bestimmung von anti-hTg-Autoantikörpern nicht zu den gewünschten Ergebnissen führt, nämlich ein Bestimmungsverfahren zu schaffen, das den Anteil an denjenigen anti-hTg-Autoantikörpern ermittelt, die tatsächlich mit dem Krankheitsgeschehen einer Autoimmunerkrankung verknüpft sind. Der Grund für die unbefriedigenden Ergebnisse bei einer Verwendung von monoklonalen anti-Tg-Antikörpen liegt vermutlich darin, daß die einzelnen Epitope, die an der anti-hTg-Autoantikörperbildung beteiligt sind, auf dem sehr großen hTg-Molekül sich gegenseitig so wenig beeinflussen, daß in kompetitiven Bestimmungsverfahren unter Verwendung monoklonaler anti-hTg-Antikörper nur Bruchteile der tatsächlich im Patientenserum vorhandenen polyklonalen anti-hTg-Autoantikörper erfaßt werden, und daß diese Bruchteile das gesamte Krankheitsgeschehen nicht adequat repräsentieren.

Versuche, anstelle von monoklonalen anti-hTg-Antikörpern tierischen Ursprungs monoklonale oder rekombinante humane anti-hTg-Antikörper zu verwenden (vgl. GB-A-2 265 713), ändern diese Problematik nicht grundsätzlich.

Es zeigt sich somit, daß die Bestimmung von anti-hTg-Autoantikörpern nach dem Muster existierender kompetitiver Bestimmungsverfahren nicht zuverlässig und mit ausreichender Empfindlichkeit möglich ist.

Der vorliegenden Erfindung liegt daher die Aufgabe zugrunde, kompetitive Bestimmungsverfahren von Antikörpern, insbesondere kompetitive Bestimmungsverfahren zur Bestimmung von anti-hTg-Autoantikörpern, so auszugestalten, daß derartige Autoantikörper auch dann mit hoher Genauigkeit und Zuverlässigkeit bestimmt werden können, wenn diese Autoantikörper gegen Antigene mit einer Vielzahl von relevanten Epitopen ausgebildet werden und als Population polyklonaler Antikörper vorliegen.

Diese Aufgabe wird im Rahmen der Bestimmung von anti-hTg-Autoantikörpern in Patientenseren gemäß Patentanspruch 1 dadurch gelöst, daß man als spezifisches Bindungsreagenz polyklonale humane Autoantikörper gegen Thyreoglobulin, und zwar insbesondere affinitätsgereinigte polyklonale humane IgG-Autoantikörper gegen Thyreoglobulin aus Seren menschlicher Patienten, die an einer Schilddrüsen-Autoimmunerkrankung leiden, verwendet.

Besonders vorteilhafte Arten der erfindungsgemäßen Verwendung von polyklonalen anti-hTg-Autoantikörpern sind den Unteransprüchen 2 bis 7 zu entnehmen.

Die vorliegende Erfindung betrifft ferner einen Reagenziensatz zur immunologischen Bestimmung von anti-hTg-Autoantikörpern in Patientenseren gemäß Anspruch 8, der im wesentlichen dadurch gekennzeichnet ist, daß er neben üblichen Bestandteilen eines derartigen Reagenziensatzes affinitätsgereinigte anti-hTg-Autoantikörper (eine affinitätsgereinigte anti-hTg-IgG-Präparation) enthält.

Wenn im Rahmen der vorliegenden Anmeldung davon gesprochen wird, daß polyklonale humane Autoantikörper gegen Thyreoglobulin als "spezifisches Bindungsreagenz" verwendet werden, so bedeutet daß, daß sie als Reaktionspartner in einem immunologischen Bestimmungsverfahren eingesetzt werden, bei dem eine spezifische immunologische Reaktion zwischen Antigen und Antikörper(n) genutzt wird. "Spezifisches Bindungsreagenz" wird nicht im Sinne von "Binder für Analyten" verwendet, da als "Analyten" die zu bestimmenden Antikörper anzusehen wären, mit denen die erfindungsgemäß zu verwendenden polyklonalen humanen Autoantikörper gegen Thyreoglobulin als Kompetitoren um Bindungsstellen des Antigens Thyreoglobulin konkurrieren.

Die vorliegende Erfindung beruht auf der Erkenntnis, daß es möglich ist, anti-hTg-Autoantikörper im Serum eines Patienten sehr zuverlässig nachzuweisen, wenn man nicht, wie auf dem Gebiet der industriellen Herstellung von Reagenziensätzen für immunologische Bestimmungsverfahren bisher üblich, mit Antikörpern (polyklonalen oder monoklonalen anti-hTg-Antikörpern) tierischen Ursprungs arbeitet, sondern mit polyklonalen humanen Autoantikörpern, die direkt aus dem Serum von Patienten gewonnen wurden, die an der nachzuweisenden Schilddrüsen-Autoimmunerkrankung nachweislich leiden. Im Rahmen der vorliegenden Erfindung können dabei generell derartige polyklonale humane Autoantikörper in Form eines Serums eines einzelnen Patienten mit den typischen Symptomen der nachzuweisenden Schilddrüsen-Autoimmunerkrankung eingesetzt werden, es ist jedoch bevorzugt, als Quelle für die polyklonalen humanen Autoantikörper die vereinigten ("gepoolten") Seren einer Mehrzahl derartiger Patienten zu verwenden, um zu gewährleisten, daß das verwendete Ausgangsserum eine möglichst vollständige Population aller Autoantikörper enthält, die bei Menschen im Zusammenhang mit der nachzuweisenden Krankheit gebildet werden können. Ferner werden im Falle der Bestimmung von anti-hTg-Autoantikörpern die polyklonalen humanen Autoantikörper vorzugsweise nicht so eingesetzt, daß alle in einem derartigen Serum vorhandenen IgG-Autoantikörper eingesetzt werden, sondern die polyklonalen humanen Autoantikörper werden vorzugsweise nach einer Affinitätsreinigung unter Verwendung einer hTg-Affinitätsmatrix verwendet, so daß die als spezifisches Bindungsreagenz eingesetzte Immunglobulinfraktion im wesentlichen nur anti-hTg-Autoantikörper umfaßt.

Wenn im Rahmen der vorliegenden Anmeldung von einer Verwendung von Thyreoglobulin oder humanem Thyreoglobulin zum Zwecke der Affinitätsreinigung oder als Reagenz in einem immunologischen Bestimmungsverfahren gesprochen wird, so ist damit in erster Linie ein übliches humanes Thyreoglobulin gemeint, wie es von verschiedenen Herstellern im Handel erhältlich ist, zum Beispiel von der britischen Firma Scipac. Da sich in jüngerer Zeit jedoch gezeigt hat, daß an der Entstehung von Autoantikörpern bei Schilddrüsenautoimmunerkrankungen ganz bestimmte Thyreoglobutinfraktionen überdurchschnittlich beteiligt sein können (Ali M. Saboori, Noel R. Rose, Rudolf C. Kuppers, Wayne G. Butscher, Herbert S. Bresler und C. Lynne Burek, in: Clinical Immunology and Immunopathology, Vol.72, No.1, S.121-128, 1994; A. Gardas, in: Autoimmunity, 1991, S. 331-336), liegt es ausdrücklich im Rahmen der vorliegenden Erfindung, sowohl zur Affinitätsreinigung als auch als Reagenz im Bestimmungsverfahren kein vollständiges Thyreoglobulinpräparat zu verwenden, sondern nur eine für Autoimmunerkrankungen besonders wichtige Fraktion des humanen Thyreoglobulins, zum Beispiel die in der genannten Literaturstelle als Protein Peak 1 bezeichnete Proteinfraktion, die einem Thyreoglobulin mit einem stark verminderten Jodierungsgrad entspricht, oder eine andere Fraktion des hTg, für die nachgewiesen werden kann, daß sie zum Beispiel bei an einer Hashimoto-Thyreoiditis Erkrankten im Vergleich zu Gesunden in erhöhtem Maße auftritt. Durch die Verwendung einer derartigen Thyreoglobulinfraktion anstatt eines vollständigen Thyreoglobulinpräparats können gegebenenfalls noch einmal weitere Vorteile im Hinblick auf die Selektivität und/oder Empfindlichkeit und Spezifität des Bestimmungsverfahrens erhalten werden.

Nachfolgend wird die erfindungsgemäße Bestimmung von humanen anti-hTg-Autoantikörpern unter Bezugnahme auf zwei Figuren und zwei Meßkurven sowie zwei Anwendungsbeispiele in weiteren Einzelheiten erläutert.

In den Figuren zeigen:
- Figur 1: das Verfahrensprinzip einer ersten Variante einer anti-hTg-Autoantikörperbestimmung unter Verwendung einer affinitätsgereinigten polyklonalen humanen IgG-Fraktion, die als immobilisierter spezifischer Binder für das in markierter Form eingesetzte hTg dient;
- Figur 2: das Verfahrensprinzip einer zweiten Variante einer anti-hTg-Autoantikörperbestimmung unter Verwendung einer affinitätsgereinigten polyklonalen humanen IgG-Fraktion, die in markierter Form als spezifischer Binder für das in imobilisierter Form eingesetzte hTg verwendet wird;
- Figur 3: eine typische Meßkurve mit zugehöriger Meßwerttabelle für eine Bestimmung von anti-hTg-Autoantikörpern bei einem Bestimmungsverfahren gemäß Ausführungsbeispiel 1; und
- Figur 4: eine typische Meßkurve mit zugehöriger Meßwerttabelle für eine Bestimmung von anti-hTg-Autoantikörpern bei einem Bestimmungsverfahren gemäß Ausführungsbeispiel 2.

Durch die Verwendung von polyklonalen humanen anti-hTg-Autoantikörpern als spezifische Bindungsreagenzien lassen sich kompetitive immunologische Bestimmungsverfahren verwirklichen, bei denen die als spezifische Bindungsreagenzien zugesetzten polyklonalen humanen Autoantikörper mit den in der Patientenprobe nachzuweisenden Autoantikörpern konkurrieren.

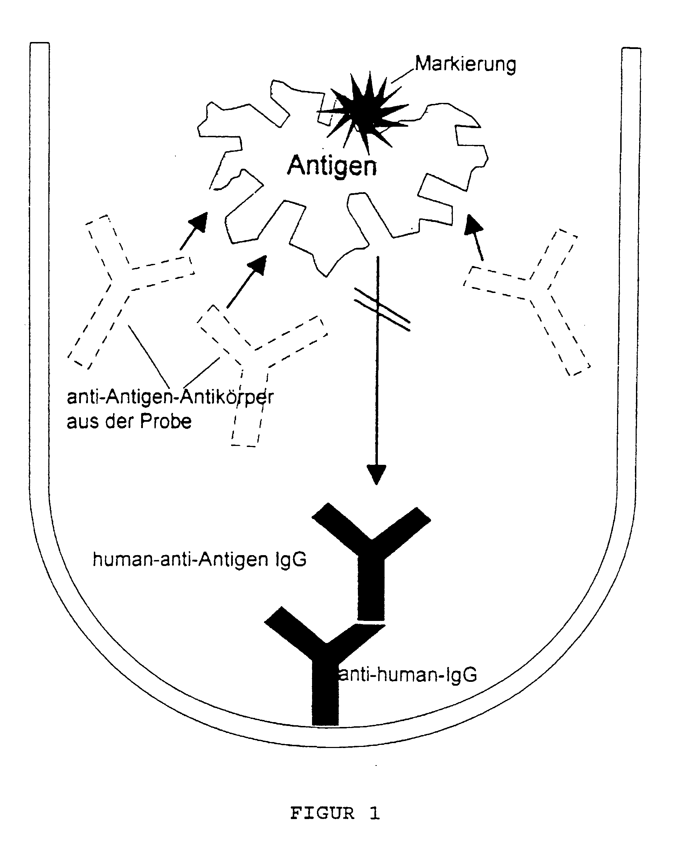

Insbesondere können die Bestimmungsverfahren so durchgeführt werden, daß man (vgl. Figur 1) gemäß einer Variante die polyklonalen humanen anti-hTg-Autoantikörper, und zwar insbesondere in Form einer affinitätsgereinigten anti-hTg-IgG-Präparation, an eine feste Trägerphase, insbesondere an die Wand eines Teströhrchens, bindet, und daß man als weiteres Reagenz ein markiertes humanes Thyreoglobulin, zum Beispiel ein radiojodiertes humanes Thyreoglobulin, einsetzt. In Abwesenheit von anti-hTg-Autoantikörpern, und zwar insbesondere in Abwesenheit von ursächlich mit der nachzuweisenden Schilddrüsenerkrankung in Verbindung stehenden hTg-Autoantikörpern, wird das markierte Thyreoglobulin vollständig beziehungsweise zu einem für eine Blindprobe charakteristischen Prozentsatz gebunden. Sind in der Patientenprobe anti-hTg-Autoantikörper der nachzuweisenden Art vorhanden, kommt es zu einer Konkurrenz der in das Verfahren als spezifisches Bindungsreagenz eingesetzten Antikörper und der Antikörper im Patientenserum, die sich dadurch äußert, daß die Bindung des markierten Thyreoglobulins an die Festphase vermindert ist, was nach einer Trennung fest-flüssig dazu führt, daß weniger Markierung an der Festphase gefunden wird.

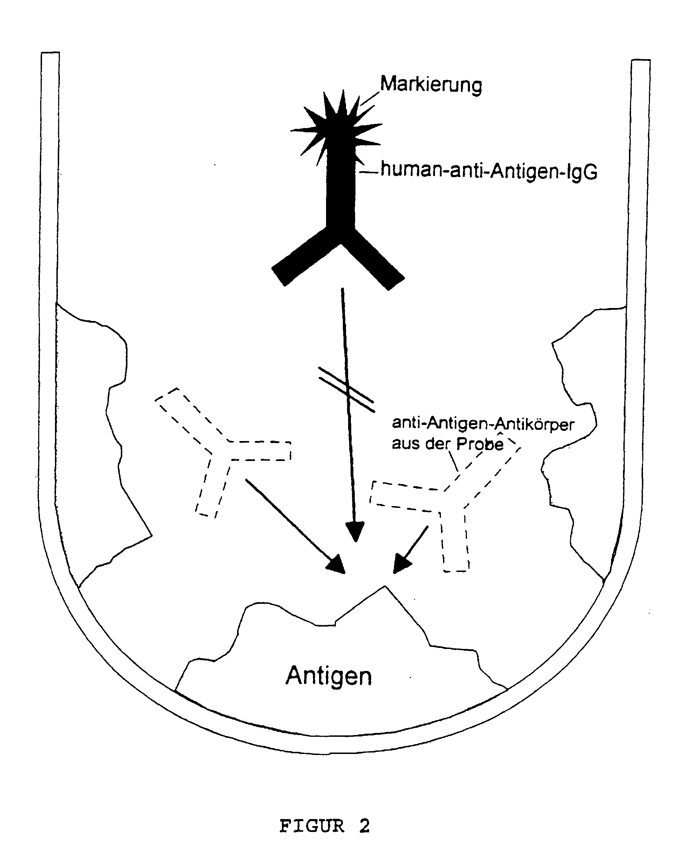

Gemäß einem anderen, grundsätzlich ebenfalls an sich bekannten Verfahrensprinzip (Figur 2) kann auch so vorgegangen werden, daß man das Antigen, das heißt humanes Thyreoglobulin (oder eine Fraktion davon, s.o.), an einer Festphase immobilisiert und daß man mit einem Tracer in Form einer markierten polyklonalen humanen antihTg-Autoantikörperfraktion arbeitet. Auch in diesem Falle führt die Anwesenheit der zu bestimmenden Autoantikörper in einer Patientenprobe dazu, daß die Bindung der Markierung an die feste Phase vermindert wird.

Auch wenn in den Beispielen der vorliegenden Anmeldung als Marker oder Label für das Tracermolekül stets ein lod-Radioisotop verwendet wird, versteht es sich für den Fachmann, daß die Natur des verwendeten Labels nicht kritisch ist und beliebige andere geeignete Label für die Tracerherstellung ausgewählt und verwendet werden können.

Die Festphase kann auch die Wandung einer Mikrotiterplatte sein oder eine andere an sich bekannte grobdisperse oder feindisperse Festphase, wobei die Auswahl der geeigneten Festphase primär anhand von praktischen Erwägungen für den speziellen Anwendungsfall getroffen werden kann.

Wie die nachfolgenden Versuche belegen, hat sich überraschend gezeigt, daß unter Verwendung von polyklonalen humanen anti-hTg-Autoantikörpern als spezifisches Bindungsreagenz eine zuverlässige anti-hTg-Autoantikörperbestimmung in humanen Seren nach kompetitiven Bestimmungsverfahren möglich ist.

Die Verwendung von polyklonalen humanen anti-hTg-Autoantikörpern als spezifisches Bindungsreagenz hat vermutlich den Vorteil, daß in einem derartigen polyklonalen spezifischen Bindungsreagenz Bindungspartner für alle möglichen Epitope am hTg-Molekül vorhanden sind, die mit der krankheitsbedingten Entwicklung von anti-hTg-Autoantikörpern zu tun haben. Damit wird aber sichergestellt, daß jegliches Auftreten von derartigen anti-hTg-Autoantikörpern in einem Patientenserum, zu einem nachweisbaren Effekt führt, wobei naturgemäß der Effekt mit der Menge und/oder der Anzahl der einzelnen Autoantikörpertypen in einem Patientenserum steigt. Der für den Fall einer Verwendung von monoklonalen Antikörpern als spezifisches Bindungsreagenz mögliche Fall, daß nämlich die spezielle Bindung zwischen monoklonalem Antikörper und Antigen durch eine bestimmte Art von Autoantikörpern aus einem Patientenserum nicht gestört wird und derartige Autoantikörper somit nicht erfaßt werden, ist bei dem erfindungsgemäßen Verfahren nicht zu erwarten. Durch die Verwendung von Patientenseren mit dem für eine bestimmte Erkrankung typischen Satz von Autoantikörpern, die als Antikörperpopulation eine Art Fingerprint-Verteilung darstellen, die mit einem bestimmten Erkrankungszustand korreliert werden kann, wird außerdem die für ein derartiges Fingerprintmuster optimal mögliche Spezifität erhalten, und unspezifische Nebeneffekte können durch eine geeignete Standdardisierung eliminiert werden.

Bei Verwendung von polyklonalen humanen anti-hTg-Autoantikörpern besteht eine ausreichende Wahrscheinlichkeit dafür, daß - auch bei Immobilisierung oder Markierung dieser Antikörper oder des zugehörigen Antigens - alle relevanten Epitope des hTg für eine Bindung mit allen relevanten Autoantikörpertypen der Population zur Verfügung stehen, so daß jeder einzelne Antikörpertyp der Population einen Beitrag zum Gesamt-Meßergebnis leisten kann, dessen klinische Relevanz dadurch steigt.

Voraussetzung für die vorliegende Erfindung war dabei jedoch noch die überraschende Erkenntnis, daß es in der Praxis kein ernsthaftes Problem darstellt, mit aus Seren von erkrankten humanen Spendern gewonnenen anti-hTg-Autoantikörpern zu arbeiten statt, wie bisher üblich, mit Antikörpern, die durch Immunisierung von Tieren gewonnen wurden oder mit entsprechenden monoklonalen Antikörpern. Es zeigte sich überraschend, daß anti-hTg-Autoantikörper in Seren von Patienten mit Hashimoto-Thyreoiditis in solchen Mengen vorhanden sind, daß eine relativ geringe Menge an Spenderserum ausreicht, den Bedarf für die Herstellung einer großen Menge von beschichteten Teströhrchen zu sichern. So lassen sich beispielsweise unter Verwendung von 10 ml Patientenserum mit einem Gehalt von z. B. 10.000 U/ml anti-Tg (bestimmt im HENNINGtest® anti-Tg der Anmelderin) 5 mg affinitätsgereinigte anti-hTg-Autoantikörper gewinnen, die für etwa 25.000 Einzelbestimmungen ausreichen. Ferner hat sich überraschenderweise gezeigt, daß dann, wenn man mit gepoolten Seren einer ausreichenden, relativ kleinen Anzahl von verschiedenen Erkrankten arbeitet, für die beabsichtigte Verwendung sehr gut geeignete polyklonale humane Autoantikörper mit weitgehend identischen Bindungseigenschaften erhalten werden, wobei von Charge zu Charge gegebenenfalls zu beobachtende Reaktivitätsfluktuationen durch die vorgesehenen üblichen Eichungsschritte leicht berücksichtigt werden können.

Bedenken hinsichtlich einer ausreichenden Verfügbarkeit und/oder Reproduzierbarkeit und Qualitätskonstanz der spezifischen Bindungsreagenzien sind somit bei Bestimmungsverfahren unter Verwendung von polyklonalen humanen anti-hTg-Autoantikörpern nicht gerechtfertigt. Derartige Bedenken könnten - im Sinne eines Vorurteils - als Erklärung dafür dienen, daß gegenwärtig in den kommerziellen Bestimmungsverfahren nie mit humanen IgG-Fraktionen als spezifischer Bindungspartner gearbeitet wird und daß es auch nicht möglich war, in der Literatur einen Vorschlag zu finden, polyklonale humane IgG-Fraktionen als spezifische Bindungspartner zur Herstellung kommerzieller Reagenziensätze für immunologische Bestimmungsverfahren zur Bestimmung von anti-hTG-Autoantikörpern in Patientenseren zu verwenden.

Nachfolgend wird die vorliegende Erfindung anhand von zwei Ausführungsbeispielen für die beiden in den Figuren erläuterten Bestimmungsverfahrenstypen von anti-hTg-Autoantikörpern noch näher erläutert.

### Ausführungsbeispiele

### Materialien

Die in sämtlichen Ausführungsbeispielen als Festphasen verwendeten Röhrchen sind Röhrchen der Firma Nunc, Maxisorp-Qualität 75 x 12 mm mit Sterneinsatz.

Die erwähnten Pufferlösungen sind:

| | |
|---|---|
| Puffer A | phosphatgepufferte Salzlösung (PBS), 0,1 % Tween 20 |
| Puffer B | 10 mM TRIS HCI, 10 mM NaCl, pH 7,8 |
| Puffer C | 20 mM Natriumphosphat, pH 7,0 |
| Puffer D | 25 mM Zitronensäure |
| Puffer E | 0,5 M Natriumphosphat, pH 8,0 |
| Puffer F | 20 mM Natriumphosphat 1 % Rinderserumalbumin (BSA; Sigma) 3 % kristallisationsverzögerter Sorbitsirup (Karion FP; Merck), pH 7,5 |

### Serumproben:

Serumproben von Patienten mit den Indikationen Hashimoto-Thyreoiditis, Autoimmun-Thyreoiditis und Morbus Basedow (Graves Disease) wurden aus verschiedenen deutschen Kliniken erhalten. Sie dienten zur Herstellung der Reagenzien bzw. als Testseren. Serumproben, die als Kontrollproben verwendet werden, sind Seren von Personen, die ohne Anzeichen irgendwelcher Infektionen, Autoimmun- oder allergischer Erkrankungen waren.

Das als Vergleichsverfahren angewandte Bestimmungsverfahren gemäß HENNINGtest anti-Tg ist ein kommerzielles Bestimmungsverfahren und in Form eines Reagenziensatzes mit Arbeitsanleitung von der Firma BRAHMS Diagnostica GmbH, Berlin zu erhalten.

Mit Jod¹²⁵ markiertes humanes Thyreoglobulin wurde dem genannten handelsüblichen HENNINGtest anti-Tg entnommen.

### Affinitätsreinigung von anti-hTg-Autoantikörpern aus Seren von Patienten mit Hashimoto-Thyreoiditis zur Reagenzienherstellung

a) Herstellung einer Affinitätsmatrix
   20 mg humanes Thyreoglobulin (Sipac, GB) werden in 7 ml Puffer C gelöst und mit 100 ml Natriumperiodat (Fluka) versetzt. Nach einer 15-minütigen Inkubation wird die Lösung mittels NAP 25-Säulen (Pharmacia) entsalzen und anschließend mit 10 ml Carbolink-Material (Pierce, USA) vermengt. Nach einer 20-stündigen Inkubation bei 4 °C wird das Material in eine 10 ml Glassäule (Biorad) gefüllt und mit Puffer C gewaschen.
b) Affinitätsreinigung der humanen anti-Tg-Autoantikörper aus Seren von Hashimoto-Patienten
   Je 1 ml Serum von 10 Patienten mit Hashimoto-Thyreoiditis werden gemischt (finaler anti-hTg-Gehalt entsprechend einer Bestimmung mit HENNINGtest® anti-Tg der Anmelderin: 5000 U/ml) und unter Verwendung einer Schlauchpumpe bei einer Flußgeschwindigkeit von 1 ml/min für 120 min im Kreislauf über die Affinitätsmatrix gepumpt. Der Säulenausfluß wird kontinuierlich auf Absorption bei 280 nm vermessen. Anschließend wird die Säule mit 50 ml Puffer C gewaschen.
   Die Elution der humanen anti-hTG-Autoantikörper erfolgt durch Waschen der Säule mit Puffer D. Die humanen anti-hTg-Autoantikörper (Volumen nach Elution: 4 ml) werden mit 4 ml Puffer E vermischt und bis zur weiteren Verwendung bei 4 °C gelagert.

### Ausführungsbeispiel 1: Bestimmung von anti-hTg-Autoantikörpern unter Verwendung eines radiojodierten Thyreoglobins als Tracer (Verfahrensvariante HD-R):

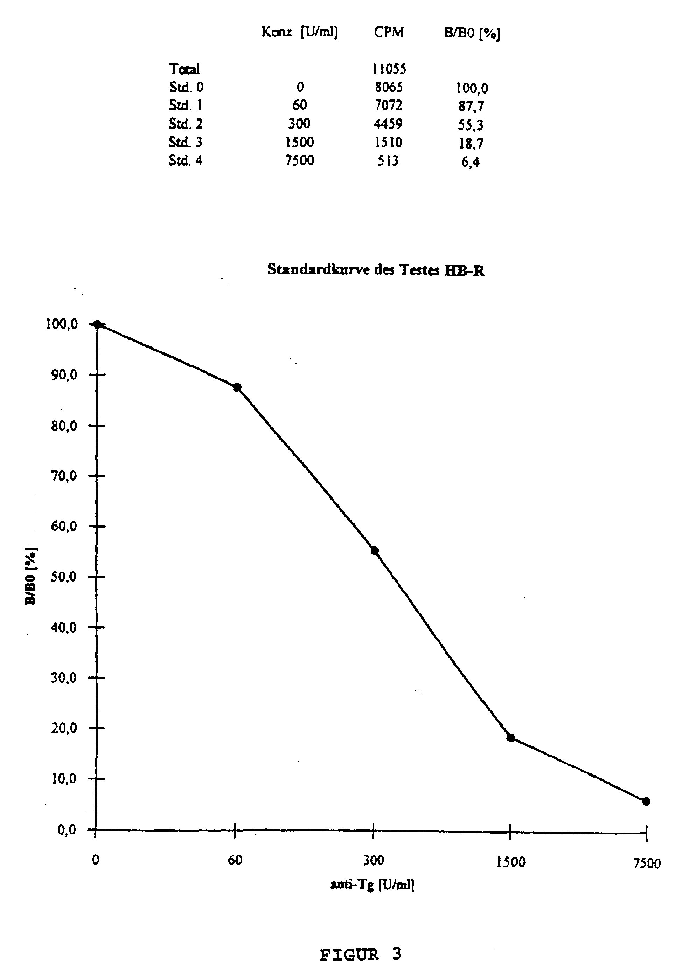

a) Herstellung von Teströhrchen mit humanen Antikörpern von Patienten mit Hashimoto-Thyreoiditis ("HD-R Röhrchen")
   Zuerst wurden Röhrchen hergestellt, die zur Bindung der humanen Antikörper mit anti-human-IgG beschichtet wurden. Dazu wurden Ziegen-anti-human-IgG (Bestell-Nr. 3AG474, Grade 2, Firma Scantibodies, USA) in Puffer B auf eine Endkonzentration von 6,7 µg/ml verdünnt. 300 µl dieser Lösung wurden in Teströhrchen (Maxisorp-Röhrchen der Fa. Nunc) pipettiert und für 20 h bei Raumtemperatur inkubiert. Anschließend wurde der Röhrcheninhalt dekantiert, und die Röhrchen wurden komplett mit Puffer F befüllt. Nach einer 2-stündigen Inkubation wurde der Röhrcheninhalt dekantiert, und die Röhrchen wurden unter Vakuum getrocknet. Die Lagerung der fertigen Röhrchen ("AHI-Röhrchen") erfolgte bei 4 °C.
   Die wie oben beschrieben affinitätsgereinigten humanen anti-hTg-Autoantikörper wurden mit Puffer A auf eine Proteinkonzentration von 2 µg/ml verdünnt, und von dieser Lösung wurden je 100 µl in die mit anti-human-IgG- beschichteten Röhrchen (AHI-Röhrchen) pipettiert und 2 Stunden unter Schütteln (bei 320 Umdrehungen/min) bei Raumtemperatur inkubiert. Dann wird zur Absättigung restlicher freier anti-human-IgG-Bindungsstellen eine Lösung mit 1 mg/ml humanem IgG in Puffer A pipettiert und weitere 2 h unter Schütteln inkubiert. Anschließend wurden die Röhrchen zweimal mit je 2 ml PBS gewaschen und bis zur weiteren Verwendung bei 4 °C gelagert.
b) Durchführung der Bestimmung
   Es wurde nach folgendem Inkubationsschema verfahren:
   1. Verdünne zur Bereitung der Proben Humanserum der Kontrollgruppen, der Patienten bzw. der Kalibratoren im Verhältnis 1 + 20 (ein Teil Serum, 20 Teile Puffer) mit Puffer A.
   2. Pipettiere 100 µl der Probe in die mit Antikörpern von Patienten mit Hashimoto-Thyreoiditis beschichteten Röhrchen (HD-R-Röhrchen).
   3. Pipettiere 100 µl eines radiojodierten humanen Tg (Bestandteil des kommerziellen HENNINGtest® anti-Tg Assays).
   4. Inkubiere 2 Stunden unter Schütteln bei Raumtemperatur.
   5. Entferne die flüssige Phase und wasche zweimal mit je 2 ml PBS.
   6. Messe die in den Röhrchen verbliebene Radioaktivität.
c) Ergebnisse
   Der radiojodierte Thyreoglobulin-Tracer wird in Abwesenheit von anti-Thyreoglobulin-Autoantikörpern in der Probe unter den angegebenen Bedingungen auf Röhrchen, welche mit IgG von Patienten mit Hashimoto-Thyreoiditis beschichtet sind, zu 73 % gebunden. Durch steigende Mengen an anti-Tg-Autoantikörpern in den Proben erfolgt eine Reduzierung der Bindung des markierten Thyreoglobulins. Typische Meßwerte der Eichung und eine typische Meßkurve sind in Figur 3 wiedergegeben.
   41 Kontrollproben werden in dem beschriebenen Verfahren genau wie im Vergleichsverfahren HENNINGtest® anti-Tg der Firma BRAHMS Diagnostica erwartungsgemäß negativ gefunden (vgl. Tabelle 1).
   85 Seren von Patienten mit entweder Hashimoto-Thyreoiditis, Morbus Basedow oder Immunthyreoiditis wurden in dem erfindungsgemäßen Verfahren sowie in dem Vergleichsverfahren HENNINGtest® anti-Tg vermessen.
   Von den vermessenen Proben, die potentiell anti-hTg-positiv sind, werden in dem erfindungsgemäßen Verfahren 35 Proben als positiv befunden und in dem Vergleichsverfahren 32 Proben. 28 Proben werden dabei in beiden Verfahren als positiv gefunden. Die Meßergebnisse sind in Tabelle 2 zusammengestellt. Die Ergebnisse zeigen, daß die Verwendung von polyklonalen humanen anti-hTg-Antikörpern zur Bestimmung von Autoantikörpern gegen Thyreoglobulin in Patientenseren geeignet ist.

### Ausführungsbeispiel 2: Bestimmung von anti-hTg-Autoantikörper unter Verwendung von markierten affinitätsgereinigten humanen anti -hTg-Autoantikörpern aus Patientenseren (Verfahrensvariante Tg-R)

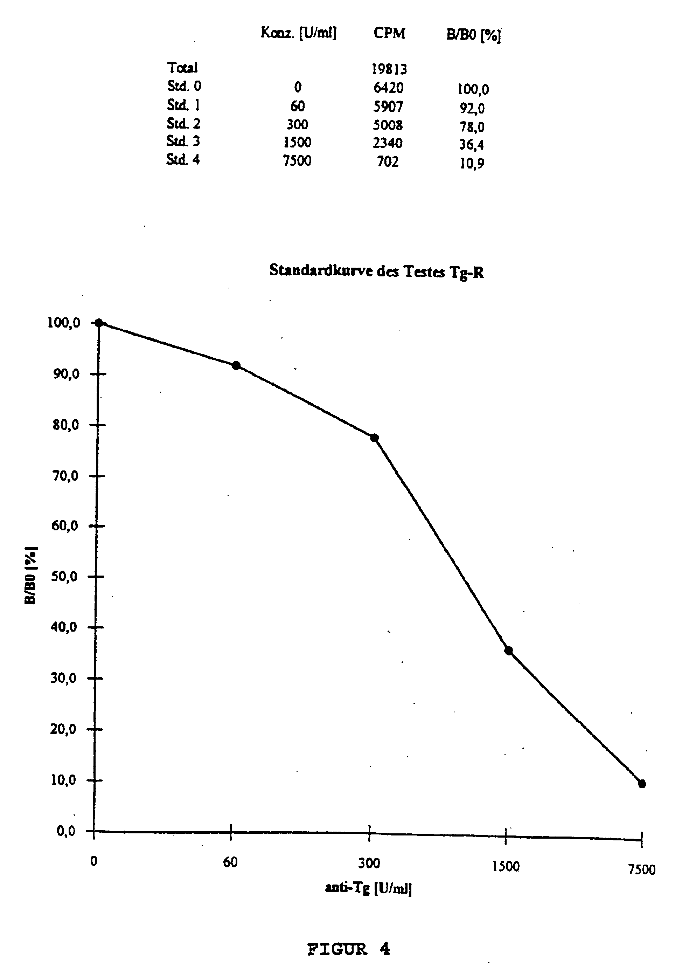

a) Radiojodierung von humanen anti-hTg-Autoantikörpern
   10 µg der wie oben beschrieben affinitätsgereinigten humanen anti-hTg-Autoantikörper aus Hashimoto-Patientenseren in einem Volumen von 50 µl werden mit 50 µl Natriumphosphatpuffer, 50 mM, pH 7,4 und 150 µCiNa¹²⁵I in 2 µl vermischt, und die Markierungsreaktion wird nach der klassischen Chloramin T-Methode durchgeführt und gestartet durch die Zugabe von 2,5 µg Chloramin T in 5 µl H₂O. Nach einer Reaktionszeit von 60 Sekunden wird die Probe mit 300 µl 50 mM Natriumphosphatpuffer pH 7,4 verdünnt und mittels einer NAP-10 Säule (Pharmacia) auf übliche Weise nach Vorschrift entsalzen. Der erhaltene markierte Antikörper wird mit 50 mM Natriumphosphatpuffer, 0,1% Tween 20, 0,2% BSA, pH 7,4 auf eine Endverdünnung von 400.000 cpm/ml verdünnt. Die Lagerung des Materials erfolgt bei 4 °C.
b) Verfahrensdurchführung
   Zur Bestimmung wird nach dem folgenden Inkubationsprotokoll vorgegangen:
   1. Verdünne zur Probenherstellung Humanserum aus Kontrollgruppen, von Patienten und Kalibratoren 1 + 20 mit Puffer A;
   2. pipettiere 100 µl der Probe in mit Thyreoglobulin beschichtete Teströhrchen
   3. pipettiere 100 µl des wie oben hergestellten radiojodierten humanen anti-hTg-Antikörpers
   4. inkubiere für zwei Stunden bei Raumtemperatur unter Schütteln
   5. trenne die flüssige Phase ab und wasche die Röhrchen zweimal mit je 2 ml PBS
   6. messe die in den Röhrchen verbliebene Radioaktivität.
c) Ergebnisse
   Der radiojodierte humane anti-hTg-Antikörper wird in Abwesenheit von anti-hTg-Antikörpem in der Probe unter den angegebenen Bedingungen auf Röhrchen, die mit humanem Thyreoglobulin beschichtet sind (Tg-R), zu 32 % gebunden. Durch steigende Mengen an anti-hTg-Antikörpern in der Probe erfolgt eine Reduzierung der Bindung des markierten humanen anti-hTg-Antikörpers (eine typische Meßkurve ist in Figur 4 dargestellt).
   10 Kontrollproben (vgl. Tabelle 1) werden in dem beschriebenen Verfahren wie in dem Vergleichsverfahren HENNINGtest® anti-Tg der Firma BRAHMS Diagnostica GmbH erwartungsgemäß negativ gefunden.
   Bei der Messung der Seren von 85 Patienten mit Hashimoto-Thyreoiditis, Morbus Basedow oder Immunthyreoiditis wurden beim Messen mittels der vorliegenden Verfahrensvariante 16 Proben als positiv ermittelt. Im Vergleichsverfahren HENNINGtest anti-Tg wurden 17 Proben als positiv ermittelt. 14 Proben der vorliegenden Variante des Bestimmungsverfahrens stimmten mit dem Vergleichsverfahren HENNINGtest® anti-Tg überein (vgl. Tabelle 2).
   Es zeigt sich somit, daß es auch möglich ist, daß aus Patientenseren gewonnene humane anti-hTg-Antikörper in markierter Form zur Bestimmung von entsprechenden Autoantikörpern gegen Thyreoglobulin in Patientenseren zu verwenden.

**Tabelle 1:**

| Messung von Kontrollseren | | | | | | |
|---|---|---|---|---|---|---|
| Kontrollgruppe | | | | | | |
| No. | Immu anti-Tg U/ml | | HD-R U/ml | | Tg-R U/ml | |
| 1 | <100 | neg. | <30 | neg. | <60 | neg. |
| 2 | <100 | neg. | <30 | neg. | <60 | neg. |
| 3 | <100 | neg. | <30 | neg. | <60 | neg. |
| 4 | <100 | neg. | <30 | neg. | <60 | neg. |
| 5 | <100 | neg. | <30 | neg. | <60 | neg. |
| 6 | <100 | neg. | <30 | neg. | <60 | neg |
| 7 | <100 | neg. | <30 | neg. | <60 | neg. |
| 8 | <100 | neg. | <30 | neg. | <60 | neg. |
| 9 | <100 | neg. | <30 | neg. | <60 | neg. |
| 10 | <100 | neg. | <30 | neg. | <60 | neg. |
| 11 | <100 | neg. | <30 | neg. | | |
| 12 | <100 | neg. | <30 | neg. | | |
| 13 | <100 | neg. | <30 | neg. | | |
| 14 | <100 | neg. | <30 | neg. | | |
| 15 | <100 | neg. | <30 | neg. | | |
| 16 | <100 | neg. | <30 | neg. | | |
| 17 | <100 | neg. | <30 | neg. | | |
| 18 | <100 | neg. | <30 | neg. | | |
| 19 | <100 | neg. | <30 | neg. | | |
| 20 | <100 | neg. | <30 | neg. | | |
| 21 | <100 | neg. | <30 | neg. | | |
| 22 | <100 | neg. | <30 | neg. | | |
| 23 | <100 | neg. | <30 | neg. | | |
| 24 | <100 | neg. | <30 | neg. | | |
| 25 | <100 | neg. | <30 | neg. | | |
| 26 | <100 | neg. | <30 | neg. | | |
| 27 | <100 | neg. | <30 | neg. | | |
| 28 | <100 | neg. | <30 | neg. | | |
| 29 | <100 | neg. | <30 | neg. | | |
| 30 | <100 | neg. | <30 | neg. | | |
| 31 | <100 | neg. | <30 | neg. | | |
| 32 | <100 | neg. | <30 | neg. | | |
| 33 | <100 | neg. | <30 | neg. | | |
| 34 | <100 | neg. | <30 | neg. | | |
| 35 | <100 | neg. | <30 | neg. | | |
| 36 | <100 | neg. | <30 | neg. | | |
| 37 | <100 | neg. | <30 | neg. | | |
| 38 | <100 | neg. | <30 | neg. | | |
| 39 | <100 | neg. | <30 | neg. | | |
| 40 | <100 | neg. | <30 | neg. | | |
| 41 | <100 | neg. | <30 | neg. | | |

**Tabelle 2:**

| Patientenseren mit folgenden Diagnosen: Hashimoto Morbus Basedow.Immunthyreoditis | | | | | | |
|---|---|---|---|---|---|---|
| No. | Henningtest anti-Tg U/ml | | HD-R U/ml | | Tg-R U/ml | |
| 1 | <100 | neg. | <30 | neg. | <60 | neg. |
| 2 | 2156 | pos | 1350 | pos. | 685 | pos. |
| 3 | 2020 | pos. | 1747 | pos. | 850 | pos. |
| 4 | 1148 | pos. | <30 | neg. | <60 | neg. |
| 5 | 221 | pos. | 422 | pos. | 174 | pos. |
| 6 | 1248 | pos. | 1434 | pos. | 820 | pos. |
| 7 | 4155 | pos. | 4208 | pos. | 4150 | pos. |
| 8 | 1030 | pos. | 870 | pos. | 794 | pos. |
| 9 | 2568 | pos. | 2940 | pos. | 1849 | pos. |
| 10 | 2338 | pos. | 2520 | pos. | 1644 | pos. |
| 11 | <100 | neg. | <30 | neg. | <60 | neg. |
| 12 | 107 | pos. | 158 | pos. | <60 | neg. |
| 13 | 4416 | pos. | 3910 | pos. | 4635 | pos. |
| 14 | 1576 | pos. | 170 | pos. | 144 | pos. |
| 15 | 972 | pos. | 973 | pos. | 620 | pos. |
| 16 | <100 | neg. | <30 | neg | 333 | pos. |
| 17 | <100 | neg. | 77 | pos. | <60 | neg. |
| 18 | 2467 | pos. | 701 | pos. | 441 | pos. |
| 19 | <100 | neg. | <30 | neg. | <60 | neg. |
| 20 | <100 | neg. | <30 | neg. | <60 | neg. |
| 21 | <100 | neg. | <30 | neg. | <60 | neg. |
| 22 | <100 | neg | <30 | neg. | 407 | pos |
| 23 | <100 | neg. | <30 | neg. | <60 | neg. |
| 24 | 963 | pos. | <30 | neg. | <60 | neg. |
| 25 | <100 | neg | <30 | neg. | <60 | neg. |
| 26 | 170 | pos | 345 | pos | 84 | pos. |
| 27 | 724 | pos. | 111 | pos | 134 | pos |
| 28 | <100 | neg. | <30 | neg. | | |
| 29 | <100 | neg | <30 | neg | | |
| 30 | <100 | neg. | <30 | neg. | | |
| 31 | <100 | neg. | <30 | neg. | | |
| 32 | 2122 | pos. | 223 | pos. | | |
| 33 | 952 | pos. | <30 | neg. | | |
| 34 | 567 | pos. | 94 | pos. | | |
| 35 | 221 | pos. | 318 | pos. | | |
| 36 | <100 | neg. | <30 | neg. | | |
| 37 | <100 | neg. | <30 | neg | | |
| 38 | 5139 | pos | 66 | pos. | | |
| 39 | <100 | neg. | 335 | neg. | | |
| 40 | <100 | neg. | <30 | neg | | |
| 41 | <100 | neg. | <30 | neg. | | |
| 42 | <100 | neg. | <30 | neg. | | |
| 43 | 322 | pos. | 84 | pos. | | |
| 44 | <100 | neg. | <30 | neg. | | |
| 45 | <100 | neg. | <30 | neg. | | |
| 46 | <100 | neg. | <30 | neg. | | |
| 47 | <100 | neg. | <30 | neg. | | |
| 48 | <100 | neg. | <30 | neg. | | |
| 49 | <100 | neg. | 46 | pos. | | |
| 50 | <100 | neg. | <30 | neg. | | |
| 51 | <100 | neg. | 139 | pos | | |
| 52 | 16443 | pos. | 17173 | pos. | | |
| 53 | 645 | pos. | 413 | pos. | | |
| 54 | <100 | neg. | <30 | neg. | | |
| 55 | <100 | neg. | <30 | neg. | | |
| 56 | <100 | neg. | <30 | neg. | | |
| 57 | <100 | neg. | <30 | neg. | | |
| 58 | <100 | neg. | <30 | neg. | | |
| 59 | <100 | neg. | <30 | neg. | | |
| 60 | <100 | neg. | <30 | neg. | | |
| 61 | <100 | neg. | <30 | neg. | | |
| 62 | <100 | neg. | <30 | neg. | | |
| 63 | <100 | neg. | 69 | pos. | | |
| 64 | <100 | neg. | <30 | neg. | | |
| 65 | <100 | neg. | <30 | neg. | | |
| 66 | 3449 | pos. | 3796 | pos. | | |
| 67 | <100 | neg. | <30 | neg. | | |
| 68 | 971 | pos. | <30 | neg. | | |
| 69 | <100 | neg. | <30 | neg. | | |
| 70 | <100 | neg. | 90 | pos. | | |
| 71 | <100 | neg. | <30 | neg. | | |
| 72 | <100 | neg. | 83 | pos. | | |
| 73 | <100 | neg. | <30 | neg. | | |
| 74 | 274 | pos. | 207 | pos. | | |
| 75 | 156 | pos | 241 | pos. | | |
| 76 | <100 | neg. | <30 | neg. | | |
| 77 | 976 | pos. | 42 | pos. | | |
| 78 | <100 | neg. | <30 | neg. | | |
| 79 | <100 | neg. | <30 | neg. | | |
| 80 | <100 | neg. | <30 | neg. | | |
| 81 | <100 | neg. | <30 | neg. | | |
| 82 | 822 | pos. | 608 | pos. | | |
| 83 | 978 | pos. | 265 | pos. | | |
| 84 | <100 | neg. | <30 | neg. | | |
| 85 | <100 | neg. | <30 | neg. | | |

## Patentansprüche

1. Verwendung von polyklonalen humanen Autoantikörpern gegen Thyreoglobulin (anti-hTg-Autoantikörpern) als spezifisches Bindungsreagenz in einem immunologischen Bestimmungsverfahren zum klinischen Nachweis von Autoantikörpern gegen Thyreoglobulin (anti-hTg-Autoantikörpern) in einer Probe einer biologischen Flüssigkeit eines Patienten.

2. Verwendung nach Anspruch 1, **dadurch gekennzeichnet, daß** die polyklonalen humanen anti-hTg-Autoantikörper in Form eines Serumspräparats von einem einzelnen oder von mehreren Patienten, die an einer Schilddrüsen-Autoimmunerkrankung, die mit dem Auftreten von anti-hTg-Autoantikörpern im Serum des Patienten verbunden ist, erkrankt sind, oder in Form einer daraus durch Affinitätsreinigung gewonnenen anti-hTg-IgG-Fraktion verwendet werden.

3. Verwendung nach Anspruch 1 oder 2, **dadurch gekennzeichnet, daß** das immunologische Bestimmungsverfahren ein kompetitives Bestimmungsverfahren ist, bei dem die zu bestimmenden anti-hTg-Autoantikörper in der Probe und die als spezifisches Bindungsreagenz eingesetzten polyklonalen humanen anti-hTg-Autoantikörper um die Bindungsstellen eines als weiteres Reagenz des Bestimmungsverfahrens eingesetzten humanen Thyreoglobulins (hTg) konkurrieren, wobei jeweils eines der genannten Reagenzien markiert ist und das jeweils andere an eine Festphase gebunden ist, und bei dem die Anwesenheit der zu bestimmenden anti-hTg-Autoantikörper in der Probe anhand der Verminderung der Bindung des markierten Reagenz an die Festphase nachgewiesen wird.

4. Verwendung nach einem der Ansprüche 1 bis 3, **dadurch gekennzeichnet, daß** die polyklonalen humanen anti-hTg-Autoantikörper in Form einer affinitätsgereinigten anti-hTg-IgG-Präparation eingesetzt werden, die an eine feste Trägerphase gebunden ist, und daß sich die Anwesenheit von anti-hTg-Autoantikörpern in der untersuchten Patientenprobe als Verminderung der Bindung eines zugesetzen markierten humanen Thyreoglobulins an die feste Trägerphase äußert.

5. Verwendung nach Anspruch 4, **dadurch gekennzeichnet, daß** als feste Trägerphase die Wand eines Teströhrchens verwendet wird und die affinitätsgereinigte anti-hTg-IgG-Präparation mit Hilfe eines unspezifischen Binders für humane IgG an die Wand des Teströhrchens gebunden ist.

6. Verwendung nach einem der Ansprüche 1 bis 3, **dadurch gekennzeichnet, daß** die polyklonalen humanen anti-hTg-Autoantikörper in Form einer affinitätsgereinigten und durch Anfügung eines Labels markierten anti-hTg-IgG-Präparation in einem Bestimmungsverfahren eingesetzt werden, bei dem als weiteres Reagenz eine mit humanem Thyreoglobulin beschichtete feste Trägerphase eingesetzt wird.

7. Verwendung nach einem der vorausgehenden Ansprüche, **dadurch gekennzeichnet, daß** die polyklonalen humanen anti-hTg-Autoantikörper in Form einer affinitätsgereinigten anti-hTg-IgG-Fraktion dadurch gewonnen werden, daß man Seren verschiedener Spenderpatienten, die an der Schilddrüsen-Autoimmunerkrankung leiden, für die das Auftreten der zu bestimmenden Autoantikörper typisch ist, zusammengibt und daraus durch Bindung an eine hTg-Affinitätsmatrix und anschließende Elution mit einem geeigneten Puffer die affinitätsgereinigte anti-hTg-IgG-Präparation gewinnt.

8. Reagenziensatz für die klinische Diagnostik zur immunologischen Bestimmung von anti-hTganti-hTg-Autoantikörpern in Patientenseren, **dadurch gekennzeichnet, daß** er neben den üblichen Komponenten Verdünnungs- und/oder Pufferlösungen, anti-Tg-Standards und anti-Tg-Nullstandard sowie ggf. Kontrollseren als zwei weitere Komponenten humanes Thyreoglobulin sowie eine affinitätsgereinigte Präparation von polyklonalen humanen Autoantikörpern gegen Thyreoglobulin (anti-hTg-Autoantikörper-Präparation) enthält, wobei entweder das humane Thyreoglobulin oder die anti-hTg-Autoantikörper-Präparation in markierter Form vorliegt und die jeweils andere der genannten beiden Komponenten als Beschichtung auf der Innenwand eines Teströhrchens vorliegt.

## Claims

1. Use of polyclonal human autoantibodies against thyroglobulin (anti-hTg autoantibodies), as a specific binding reagent in an immunological assay for the clinical detection of autoantibodies against thyroglobulin (anti-hTg autoantibodies) in a sample of a biological fluid of a patient.

2. Use according to Claim 1, **characterized in that** the polyclonal human anti-hTg autoantibodies are used in the form of a serum preparation from an individual patient or from several patients who is or are suffering from a thyroid autoimmune disease which is associated with the occurrence of anti-hTg autoantibodies in the serum of the patient, or in the form of an anti-hTg-IgG fraction obtained therefrom from by affinity purification.

3. Use according to Claim 1 or 2, **characterized in that** the immunological assay is a competitive assay in which the anti-hTg autoantibodies to be determined and present in the sample and the polyclonal human anti-hTg autoantibodies used as a specific binding reagent compete for the binding sites of a human thyroglobulin (hTg) used as a further reagent of the assay, one of the stated reagents in each case being labelled and the other being bound to a solid phase, and in which the presence of the anti-hTg autoantibodies to be determined and present in the sample is detected on the basis of the reduction of the binding of the labelled reagent to the solid phase.

4. Use according to any of Claims 1 to 3, **characterized in that** polyclonal human anti-hTg autoantibodies are used in the form of an affinity-purified anti-hTg-IgG preparation which is bound to a solid carrier phase, and that the presence of anti-hTg autoantibodies in the patient's sample investigated manifests itself as a reduction of the binding of an added labelled human thyroglobulin to the solid carrier phase.

5. Use according to Claim 4, **characterized in that** the wall of a test tube is used as the solid carrier phase and the affinity-purified anti-hTg-IgG preparation is bound to the wall of the test tube with the aid of an unspecific binder for human IgG.

6. Use according to any of Claims 1 to 3, **characterized in that** the polyclonal human anti-hTg autoantibodies are used in the form of an affinity-purified anti-hTg-IgG preparation marked by attachment of a label, in an assay in which a solid carrier phase coated with human thyroglobulin is used as a further reagent.

7. Use according to any of the preceding claims, **characterized in that** the polyclonal human anti-hTg autoantibodies are obtained in the form of an affinity-purified anti-hTg-IgG fraction by combining sera of different donor patients who suffer from the thyroid autoimmune disease for which the occurrence of the autoantibodies to be determined is typical, and the affinity-purified anti-hTg-IgG preparation is obtained therefrom by binding to an hTg affinity matrix and subsequent elution with a suitable buffer.

8. Reagent kit for the clinical diagnosis for the immunological determination of anti-hTg autoantibodies in patients' sera, **characterized in that**, in addition to the usual components comprising dilution and/or buffer solutions, anti-Tg standards and anti-Tg zero standards and optionally control sera, it contains human thyroglobulin and an affinity-purified preparation of polyclonal human autoantibodies against thyroglobulin (anti-hTg autoantibody preparation) as two further components, either the human thyroglobulin or the anti-hTg autoantibody preparation being present in labelled form and the other component of the two stated components being present as a coating on the inner surface of a test tube.

## Revendications

1. Utilisation d'auto-anticorps humains polyclonaux dirigés contre la thyréoglobuline (auto-anticorps anti-hTg) comme réactif de liaison spécifique dans un procédé de détermination immunologique, en vue de la détection clinique d'auto-anticorps dirigés contre la thyréoglobuline (auto-anticorps anti-hTg) dans un échantillon d'un fluide biologique d'un patient.

2. Utilisation selon la revendication 1, **caractérisée en ce que** les auto-anticorps anti-hTg humains polyclonaux sont utilisés sous la forme d'une préparation de sérum provenant d'un seul patient ou de plusieurs patients qui souffrent d'une maladie auto-immune de la thyroïde, associée à l'apparition d'auto-anticorps anti-hTg dans le sérum du patient, ou sous la forme d'une fraction IgG anti-hTg obtenue dans ce ou ces sérums par purification par affinité.

3. Utilisation selon la revendication 1 ou 2, **caractérisée en ce que** le procédé de détermination immunologique est un procédé de détermination par compétition, dans lequel les auto-anticorps anti-hTg à déterminer, présents dans l'échantillon, et les auto-anticorps anti-hTg humains polyclonaux utilisés comme réactif de liaison spécifique entrent en concurrence pour des sites de liaison d'une thyréoglobuline humaine (hTg) utilisée comme autre réactif du procédé de détermination, et dans chaque cas l'un desdits réactifs est marqué et l'autre est lié à une phase solide, et la présence des auto-anticorps anti-hTg à déterminer dans l'échantillon est détectée sur base de la diminution de la liaison du réactif marqué à la phase solide.

4. Utilisation selon l'une des revendications 1 à 3, **caractérisée en ce que** les auto-anticorps anti-hTg humains polyclonaux sont utilisés sous la forme d'une préparation d'IgG anti-hTg purifiée par affinité qui est liée à une phase porteuse solide, et **en ce que** la présence d'auto-anticorps anti-hTg dans l'échantillon de patient étudié s'exprime par une diminution de la liaison à la phase porteuse solide d'une thyréoglobuline humaine marquée que l'on ajoute.

5. Utilisation selon la revendication 4, **caractérisée en ce que** l'on utilise comme phase porteuse solide la paroi d'un tube à essai, et la préparation d'IgG anti-hTg purifiée par affinité est liée à la paroi du tube à essai à l'aide d'un ligand non spécifique pour l'IgG humaine.

6. Utilisation selon l'une des revendications 1 à 3, **caractérisée en ce que** les auto-anticorps anti-hTg humains polyclonaux sont utilisés sous la forme d'une préparation d'IgG anti-hTg purifiée par affinité et marquée par jonction à un identificateur, dans un procédé de détermination dans lequel on utilise comme autre réactif une phase porteuse solide enduite de thyréoglobuline humaine.

7. Utilisation selon l'une des revendications précédentes, **caractérisée en ce que** les auto-anticorps anti-hTg humains polyclonaux, qui présentent la forme d'une fraction d'IgG anti-hTg purifiée par affinité, sont obtenus en rassemblant des sérums de différents patients donneurs souffrant de la maladie auto-immune de la thyroïde pour laquelle l'apparition des auto-anticorps à déterminer est typique, et que la préparation d'IgG anti-hTg purifiée par affinité est obtenue à partir de là, par liaison à une matrice d'affinité hTg et ensuite élution par un tampon approprié.

8. Jeu de réactifs pour le diagnostic clinique en vue de la détermination immunologique d'auto-anticorps anti-hTg dans des sérums de patients, **caractérisé en ce qu'**en plus des composants habituels, il contient des solutions de dilution et/ou tampons, des étalons anti-Tg et des témoins anti-Tg ainsi qu'éventuellement des sérums témoins en tant que deux autres composants de la thyréoglobuline humaine, ainsi qu'une préparation d'auto-anticorps humains polyclonaux dirigés contre la thyréoglobuline (préparation d'auto-anti-corps anti-hTg) purifiée par affinité, tandis que soit la thyréoglobuline humaine, soit la préparation d'auto-anticorps anti-hTg se présente sous forme marquée, et l'autre desdits deux composants se présente sous la forme d'une couche appliquée sur la paroi intérieure d'un tube à essai.
